# EUROPEAN PATENT APPLICATION

(11) **EP 1 939 543 A2**
(43) Date of publication of application: **02.07.2008**
(21) Application number: 07024847.1
(22) Date of filing: 20.12.2007
(51) Int. Cl.: F24F 13/22

(54) **Air conditioner**

(30) Priority: 26.12.2006 JP 2006349090
(71) Applicant: Sanyo Electric Co., Ltd., Moriguchi-shi, Osaka (JP)
(72) Inventor: Nishihara, Takuro, Ota-shi, Gunma 373-0817 (JP); Fukushima, Toshio, Ota-shi, Gunma 373-0038 (JP)
(74) Representative: Glawe, Delfs, Moll

(57) **Abstract**

An air conditioner including a heat exchanger (21) for air sucked by an air blower (22), a drain pan (24) for receiving drain water dropping from the heat exchanger (21), a drain pump (27) for pumping up drain water stocked in the drain pan (24) and discharging the drain water through a drain hose (27a) to the outside, a gas-liquid contact member (41) that is disposed at the downstream side of the heat exchanger (21) in an air flowing direction and supplied with electrolytic water containing active oxygen specifies so that the electrolytic water infiltrates into the gas-liquid contact member (41) and brought into contact with the air sucked by the air blower (22) to filter the air, and a housing (20) in which the heat exchanger, the drain pan, the drain pump and the gas-liquid contact member are mounted, wherein the electrolytic water dropped form the gas-liquid contact member is guided to an electrolytic water receiver (62) which is provided separately from the drain pan, and the electrolytic water passed through the electrolytic water receiver (62)is made confluent with the drain water at the downstream side of the drain pump (27).

## Description

### BACKGROUND OF THE INVENTION

### 1. Field of the Invention

The present invention relates to an air conditioner in which microorganisms floating in the air (bacteria, virus, fungus, etc. (hereinafter referred to as "virus, etc.") can be removed.

### 2. Description of the Related Art

There has been known a method of generating electrolytic water containing active oxygen species such as hypochlorous acid or the like by electrolyzing water and bringing the generated electrolytic water into contact with air to decompose/remove harmful materials contained in the air, thereby purifying (filtering) the air. Furthermore, there is known a technique of supplying filtered air to an air conditioner to cool, heat or humidify air from which harmful materials are decomposed/removed, thereby supplying comfortable air into a room (see JP-A-2003-250876).

In the case of the construction that electrolytic water which has been already used for air filtering is guided to and stocked in a drain pan disposed below a heat exchanger and pumped up and discharged by a drain pump together with condensed water (drain water) generated in the heat exchanger, a part of the heat exchanger may be immersed in drain water contaminated in the electrolytic water stocked in the drain pan. In this case, if the concentration of the active oxygen specifies of the electrolytic water or the concentration of ions (for example, the concentration of chlorine ion concentration) is high, a steep pipe and a fin constituting the heat exchanger may be eroded. Furthermore, the drain pan itself may corrode when some kinds of materials are used for the drain pan.

### SUMMARY OF THE INVENTION

Therefore, an object of the present invention is to provide an air conditioner in which corrosion of a drain pan and a heat exchanger located in the drain pan can be suppressed.

In order to attain the above object, an air conditioner including a heat exchanger (21) for air sucked by an air blower (22), a drain pan (24) for receiving drain water dropping from the heat exchanger (21), a drain pump (27) for pumping up drain water stocked in the drain pan (24) and discharging the drain water through a drain hose (27a) to the outside, a gas-liquid contact member (41) that is disposed at the downstream side of the heat exchanger (21) in an air flowing direction and supplied with electrolytic water containing active oxygen specifies so that the electrolytic water infiltrates into the gas-liquid contact member (41) and brought into contact with the air sucked by the air blower (22) to filter the air, and a housing (20) in which the heat exchanger, the drain pan, the drain pump and the gas-liquid contact member are mounted, wherein the electrolytic water dropped form the gas-liquid contact member is guided to an electrolytic water receiver (62) which is provided separately from the drain pan, and the electrolytic water passed through the electrolytic water receiver (62) is made confluent with the drain water at the downstream side of the drain pump (27).

In the above air conditioner, it is preferable that the drain hose is equipped with a confluent portion (66) at which the drain water and the electrolytic water are confluent with each other, and the confluent portion (66) is provided so as to be located at the downstream side of the maximum position of the drain hose (27a) in the height direction and at a lower position than the maximum position.

In the above air conditioner, it is preferable that an opening portion (20d) is formed in at least one surface of the housing, the gas-liquid contact member is fixed to one surface of a plate-shaped member (3a) which covers the opening portion (20d), and an electrolytic unit (5) for generating the electrolytic water is provided on the other surface of the plate-shaped member.

In the above air conditioner, it is preferable that the electrolytic receiver (62) is provided to the other surface of the'plate-shaped member.

According to the present invention, the electrolytic water dropping from the gas-liquid contact member is guided to the electrolytic water receiver which is different from the drain pan, thereby preventing the electrolytic water from flowing into the drain pan. Therefore, the corrosion of the drain pan and the heat exchanger located in the drain pan can be suppressed from being corroded. Furthermore, the electrolytic water passing through the electrolytic water receiver is made confluent with the drain water at the downstream side of the drain pump and then discharged. Therefore, only one drain hose may be provided to the outside of the apparatus, so that the pipe construction can be simplified and the construction cost can be also reduced. Since the electrolytic water is discharged through this drain hose, various bacterial, etc. can be suppressed from occurring in the drain hose, and the inside of the drain hose can be kept under a clean state.

### BRIEF DESCRIPTION OF THE DRAWINGS

Fig. 1 is a diagram showing the construction of an air conditioner according to an embodiment of the present invention;
Fig. 2 is a cross-sectional view showing an indoor unit;
Fig. 3 is an exploded perspective view showing the indoor unit;
Figs. 4A and 4B are diagrams showing the construction of the main part of an air filtering unit, wherein Fig. 4A is a perspective view showing the construction of an air filtering portion, and Fig. 4B is a diagram showing the construction of an electrolytic unit; and
Fig. 5 is a systematic diagram showing flow of electrolytic water passing through the air filtering portion.

### DETAILED DESCRIPTION OF THE PREFERRED EMBODIMENT

An air conditioner according to an embodiment of the present invention will be described with reference to the accompanying drawings. In this embodiment, a four-way air-blowing type in-ceiling mount type air conditioner will be described as an example of the air conditioner.

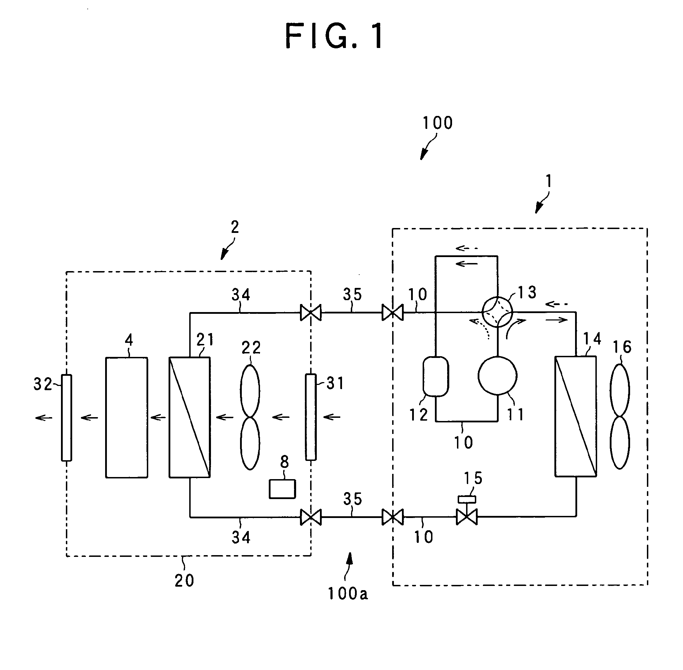

Fig. 1 is a diagram showing the construction of an air conditioner according to this embodiment. The air conditioner 100 of this embodiment is a separation type heat-pump air conditioner having an outdoor unit 1 disposed at outside of a room to be air-conditioned, and an indoor unit 2 disposed in the room to be air-conditioned.

An outdoor refrigerant pipe 10 of the outdoor unit 1 and an indoor refrigerant pipe 34 of the indoor unit 2 are connected to each other through a connection pipe 35, and the operations of the outdoor unit 1 and the indoor unit 2 are controlled by a controller 8 contained in the indoor unit 2.

As shown in Fig. 1, in the outdoor unit 1, a compressor 11 is disposed in the outdoor refrigerant pipe 11, and an accumulator 12 is connected to the suction side of the compressor 11. Furthermore, a four-way valve 13, an outdoor heat exchanger 14 and an electrically-operated expansion valve 15 are successively connected to the discharge side of the compressor 11 in this order. Furthermore, an outdoor fan 16 for blowing air to the outdoor heat exchanger 14 is disposed in the outdoor unit 1.

As shown in Fig. 1, the housing 20 has an air suction port 31 and an air blow-out port 32, and the indoor unit 2 is equipped an indoor heat exchanger 21 and an air blowing fan (air blower) 22 for making air flow from the air suction port 31 to the air blow-out port 32 in the housing 20.

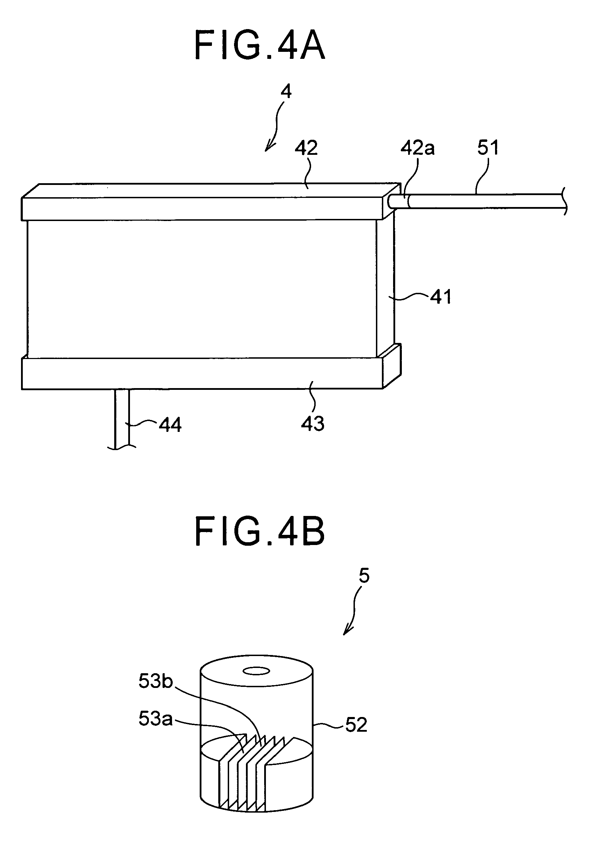

An air filtering portion 4 through which air heat-exchanged by the indoor heat exchanger 21 is passed is disposed at the downstream side of the indoor heat exchanger 21, that is, at the air blow-out port 32 side in the air flowing passage extending from the air suction port 31 to the air blow-out port 32 in the housing 20. The air filtering portion 4 has an element 41 (see Fig. 4A) in which electrolytic water containing active oxygen specifies described later is infiltrated, and the air heat-exchanged by the indoor heat exchanger 21 is brought into contact with the electrolytic water containing the active oxygen specifies in the element 41 to thereby filter the air.

The controller 8 is equipped with CPU (not shown), a control program executed by CPU, ROM for storing a control data associated with the control program, etc. , and RAM for temporarily storing programs executed by CPU and various kinds of data. Furthermore, the controller 8 is equipped with an infrared receiver for receiving an infrared signal transmitted from a remote controller (not shown) at the outside of the indoor unit 2. CPU accepts an instruction from the remote controller on the basis of the infrared signal received by the infrared receiver, reads out the control program stored in ROM according to this instruction, develops the read-out control program into RAM and executes the control program, thereby performing the overall control of the air conditioner 100.

In the air conditioner 100, by switching the four-way valve 13, the flow direction of refrigerant flowing through a refrigerant circuit 100a is switched so that cooing operation and heating operation are switched to each other. Under cooling operation, the refrigerant flows in a direction indicated by a solid-line arrow in Fig. 1, and under heating operation, the refrigerant flows in a direction indicated by a broken-line arrow of Fig. 1.

That is, under cooling operation, high-pressure refrigerant discharged from the compressor 11 passes through the accumulator 12 to the outdoor heat exchanger 14, and then the refrigerant is condensed in the outdoor heat exchanger 14 and fed to the electrically-operated expansion valve 15. The high-pressure refrigerant concerned is passed and expanded through the electrically-operated expansion valve 15, evaporated in the indoor heat exchanger 21 and then returned to the air suction side of the compressor 11. On the other hand, under heating operation, high-pressure refrigerant discharged from the compressor 11 is passed through the outdoor refrigerant pipe 10, fed to the indoor heat exchanger 21, condensed in the indoor heat exchanger 21, and then fed to the electrically-operated expansion valve 15. This refrigerant is expanded in the electrically-operated expansion valve 15, fed to the outdoor heat exchanger 14, evaporated in the outdoor heat exchanger 14, fed through the four-way valve 13 to the accumulator 12, and then returned to the suction side of the compressor 1.

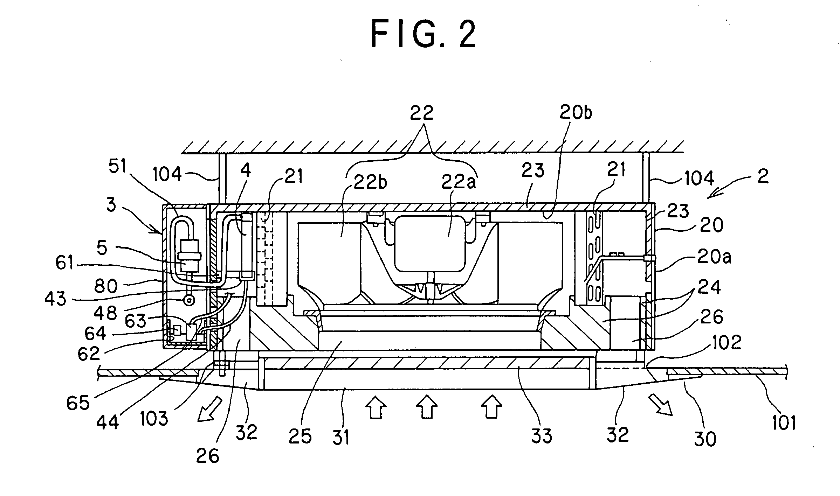

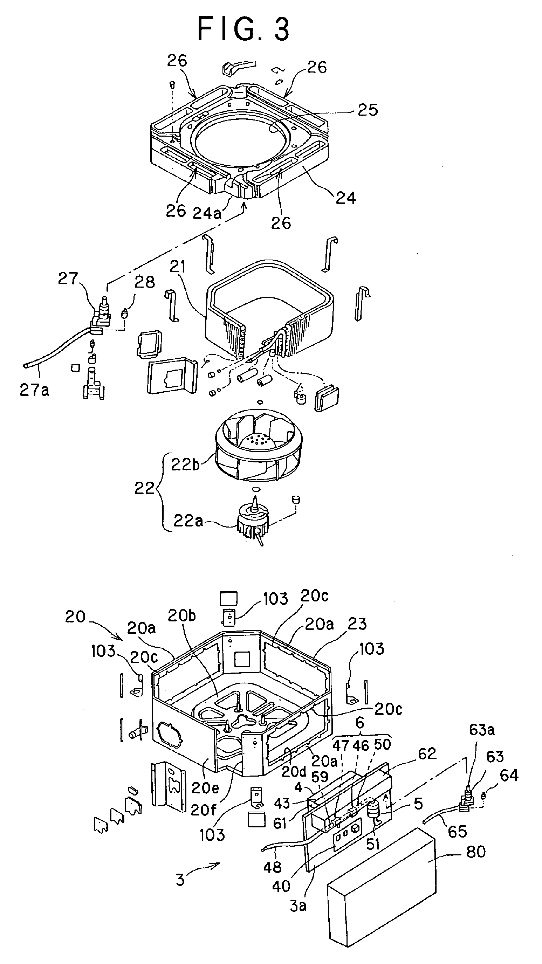

Fig. 2 is a side cross-sectional view showing a state that the indoor unit used in the air conditioner according to this embodiment is embedded in the ceiling. Fig. 3 is an exploded perspective view showing the exploded state of the indoor unit 2 while the indoor unit 2 is turned upside down.

The indoor unit 2 has a housing 2 for accommodating the indoor heat exchanger 21, the air blowing fan 22, etc., and a face panel 30 is secured to the room side of the housing 20. The face panel 30 is located at the lower side of the housing 20 under the state that the indoor unit 2 is mounted in the ceiling space as shown in Fig. 2.

The housing 20 is designed in a substantially octagonal shape so that a surface thereof at the room side (the lower surface in Fig. 2, the upper surface in Fig. 3) is open. Specifically, the housing 20 has three side plates 20a each having a substantially rectangular shape and one side plate 20e having a substantially rectangular shape. A different flat plate is interposed between the respective side plates, and the side plates and the flat plates form a substantially octagonal frame as a whole.

Knockout hole portions 20c are formed in the side plates 20a. Each knockout hole portion 20c is a substantially rectangular hole which is blocked off by one plate constituting a part of the side plate 20a, and it is pressed in so that the plate closing the hole drops off and the side plate 20a is opened as occasion demands. The opening formed by pressing in the knockout portion 20c will be referred to as "opening portion 20d".

A cut-out portion 20f for guiding the indoor refrigerant pipe 34 connected to the indoor heat exchanger 21 in the indoor unit 2, etc. is formed at one end side of the side plate 20e.

The face panel 30 is designed to be substantially rectangular in plan view, more specifically substantially square in plan view, and the opening surface and the ceiling hole 102 are covered by this face panel. The face panel 30 is provided with an air suction port 31 formed substantially at the center portion of the face panel 30 in plan view and air blow-out ports 32 which are formed in the neighborhood of the four sides of the face panel 30 so as to be elongated along the respective sides, The air blow-out ports 32 extend substantially in parallel to the side plates 20a, 20e, so that air passing through the indoor heat exchanger 21 is efficiently discharged to the room.

Furthermore, a filter 33 for removing dust contained in air flowing through the air suction port 31 into the housing 20 is mounted inside the air suction port 31, that is, at the back side of the ceiling 101. In this construction, the indoor unit 2 is designed so that air to be air-conditioned is sucked from the air suction port 31 into the housing 20, heat-exchanged in the housing 20 and then blown out from the four air blow-out ports 32 in four directions into a room to be air-conditioned.

Suspending tags 103 are secured to the four corners of the housing 20. As shown in Fig. 2, the ceiling hole 102 is formed in a substantially rectangular shape in the ceiling 101 of a building in which the indoor unit 2 is mounted, and the indoor unit 2 is embedded from the room to be air-conditioned into the ceiling hole 102 at the back side of the ceiling 101. Furthermore, suspending bolts 104 suspended from the back side of the ceiling are fixed to the suspending tags, whereby the indoor unit 2 is suspended in the ceiling space.

Next, the internal construction of the housing 20 will be described with reference to Figs. 1 to 3. As shown in Fig. 2, a heat insulating member 23 formed of foamed polystyrene is provided on the inner surfaces of the side plate 20a of the housing 20. A motor 22a is fixed to the inner surface 20b of the top plate of the housing 20, and a vane wheel 22b is secured to the shaft of the motor 22a. These units constitute the air blowing fan 22. The indoor heat exchanger 21 which is bent along the side plates 20a and 20e of the housing 20 is disposed inside the heat insulating member 23 of foamed polystyrene so as to surround the air blowing fan 22 (see Fig. 3). Air sucked from the air suction port 31 by the air blowing fan 22 is supplied to the indoor heat exchanger 21, and the air heat-exchanged by the indoor heat exchanger 21 is blown out from the respective air blow-out ports 32.

As described above, the indoor heat exchanger 21 disposed in the housing 20 is designed in the shape along the substantially octagonal frame constructed by the side plates 20a, 20e and the flat plates, and the confronting surfaces of the side plates and the flat plates are flat. A drain pan 24 of foamed polystyrene is disposed so as to be spaced from the end of the indoor heat exchanger 21 by a predetermined distance. The outer peripheral surface of the drain pan 24 substantially comes into contact with the inner surface of the housing 20. The drain pan 24 is located at the lower side of the indoor heat exchanger 21 under the mount state of the indoor unit 2 shown in Fig. 2, and mainly receives and stocks condensed water (drain water) dropping off from the indoor heat exchanger 21 under cooling operation. As shown in Fig. 3, a drain pump 27 and a float switch 28 for detecting the drain water stocked in the drain pan 24 are disposed at the position 24a corresponding to one corner of the indoor heat exchanger 21, and upon actuation of the float switch 28, the drain water stocked in the drain pan 24 is pumped up by the drain pump 27. The drain pump pumped by the drain pump 27 is discharged to the outside of the indoor unit 2 through a drainpipe (drain hose) passing through the cut-out portion 20f and extending to the outside of the housing 20. Furthermore, a connection pipe (refrigerant pipe) 35 (Fig. 1) extending from the indoor heat exchanger 21 is also passed through the cut-out portion 27f.

The drain pan 24 is provided with an air suction opening 25 and air blow-out openings 26 at the positions corresponding to the air suction port 31 and the air blow-out ports 32 of the face panel 30. As shown in Fig. 3, the air suction opening 25 is formed in a substantially circular shape in plan view at the center of the drain pan 24 which is designed in a substantially rectangular shape. Furthermore, the air blow-out openings 26 are formed along the four sides of the drain pan 24. The air blow-out openings 26 of the drain pan 2.5 are located at the positions corresponding to the flat portions of the indoor heat exchanger 21, and air is blown out from the air blow-out openings 26 through the air blow-out ports 32 to the room to be air-conditioned.

The air filtering unit 3 having the air filtering portion 4 is fitted into the opening portion 20d formed in the knockout hole portion 20c from the outside. In the air conditioner 100 according to this embodiment, the knockout hole portion 20c is opened in one side plate out of the three side plates 20a constituting the housing 20 of the indoor unit 2, the one side plate being adjacent to one corner at which the drain pump 27 is disposed, and the air filtering unit 3 is disposed.

The air filtering unit 3 is provided with a base plate (plate-shaped member) 3a for closing the opening portion 20d, and the opening portion 20d is closed by fitting the air filtering unit 3 into the opening portion 20d and fixing the base plate 3a and the side plate 20a to each other.

The base plate 3a is provided with a heat insulating member of foamed polystyrene on a surface thereof which faces the inside of the housing 20, that is, the inner surface side of the housing 20, and the air filtering portion 4 is secured so as to be spaced from the heat insulating member at a predetermined interval. The air filtering portion 4 is mounted on the basis plate 3a by a fixing bracket 61.

Furthermore, the base plate 3a is provided with an electrolytic water 5, a tap water control valve 46, a check valve 47, an electrical equipment board 40, a drain tray 62, a drain pump 63, a float switch 64, etc. According to this construction, in the air filtering unit 3, various kinds of equipment are provide to both the surfaces of the base plate 3a. Therefore, by fixing the base plate 3a to the side plate 20a, the air filtering unit can be easily secured, and also the air filtering unit 3 can be easily afterward secured to an existing indoor unit having a housing 20 on which a knockout hole portion 20c is formed.

In this embodiment, a substantially box type outer package is disposed on the housing 20 so as to cover the air filtering unit 3 from the outside of the housing 20. This outer package 80 is connected to the outside of the side plate 20a of the housing 20, and accommodates therein the respective parts of the electrolytic unit 5, the tap water control valve 46, the check valve 47, the electrical equipment board 40, the drain tray 62, the drain pump 63 and the float switch 64.

According to the above construction, the air filtering unit 3 is covered by the outer package 80, and thus a user can be prevented from carelessly touching the air filtering unit 3. In addition, by detaching the outer package 80, the electrolytic unit 5, the tap water control valve 46, the check valve 47, the electrical equipment board 40, the drain tray 62, the drain pump 63 and the float switch 64 of the air filtering unit 3 are exposed, so that maintenance can be easily performed on these units.

Under the state that the air filtering unit is fitted in the opening portion 20d, the air filtering portion 4 is located so as to be adjacent to the indoor heat exchanger 21. The air filtering portion 4 is located at the outside of the indoor heat exchanger, and thus air which is blown out by the air blowing fan 22 and passed through the indoor heat exchanger 21 is blown to the air filtering portion 4. The air is filtered (sterilized, deodorized, etc.) while passing through the air filtering portion 4, and it flows downwardly between the air filtering portion 4 and the base plate 3a and blown out from the air blow-out ports 32 formed in the face panel 30 into the room to be air-conditioned. As described above, the base plate 3a is provided with the heat insulating member, and the air passing through the air filtering portion 4 flows downwardly between the heat insulating member and the air filtering portion 4, so that the temperature variation of the air in the air filtering unit 3 can be suppressed to the minimum level.

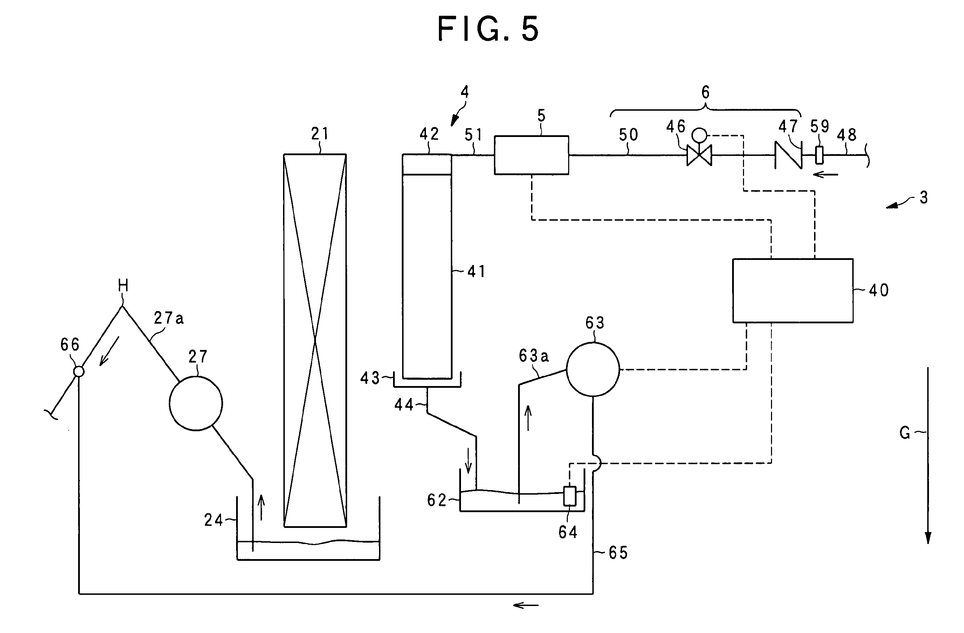

Figs. 4A and 4B show the construction of the main part of the air filtering unit 3, wherein Fig. 4A is a perspective view showing the construction of the air filtering portion 4, and Fig. 4B is a diagram showing the construction of an electrolytic unit 5. Furthermore, Fig. 5 is a diagram showing the construction of the air filtering unit 3. In Fig. 5, in order to make the understanding easy, the indoor hat exchanger 21, the drain pan 24 and the drain pump 27 are illustrated, and also a part of the filtering unit 3 is schematically shown. Furthermore, in Fig. 5, a direction indicated by an arrow G is set to the downward direction.

As shown in Fig. 4A, the air filtering portion 4 is equipped with an element having high water retentivity (gas-liquid contact member) 41, a water dispersion tray 42 disposed on the element 41 under the state that the indoor unit 2 is set, and an electrolytic water tray 43 disposed below the element 41. The element 41 comprises non-woven cloth formed of acrylic fiber, polyester fiber or the like. A material having little reactivity to electrolytic water is preferably used as the material of the element 41, and in addition to acrylic fiber and polyester fiber,polyolefintype resin(polyethylene resin, polypropylene resin or the like), vinyl chloride resin, fluorinated resin (PTFE, PFA, ETFE or the like), cellulose type material, ceramics type material or the like may be used.

In this embodiment, affinity of the element 41 to electrolytic water is enhanced by subjecting the element 41 to an affinity treatment. Accordingly, the water retentivity and wettability in the element 41 can be kept, and air introduced into the element 41 can be surely brought into contact with electrolytic water.

The water dispersion tray 42 is used to substantially uniformly dispersing onto the top surface of the element 41 electrolytic water supplied from the electrolytic unit 5 through an electrolytic water injection tube 51. A connection port 42a to which the electrolytic water injection tube 51 is connected is formed in the side surface of the water dispersion tray. Furthermore, may holes (not shown) through which electrolytic water is dropped to the element 41 and dispersed/infiltrated to the element 41 are formed in the bottom surface of the water dispersion tray 42. By dropping the electrolytic water from the water dispersion tray 42 to the element 41, the electrolytic water is uniformly supplied to the overall element 41. The electrolytic water injection tube 51 guides electrolytic water generated in the electrolytic unit 5 to the water dispersion tray 42.

When the electrolytic water supplied from the water dispersion tray 42 into the element 41 drops from the element 41, the electrolytic water tray 43 receives and stocks the electrolytic water, and a drain pipe 44 for guiding electrolytic water to the drain tray 62 is connected to the bottom surface of the electrolytic water tray 43. Both the ends of the electrolytic water tray 43 are supported and fixed to the base plate 3a by a mounting tab 61 as shown in Fig. 2. Furthermore, the element 41 is connected to the electrolytic water tray 43 by a stay (not shown), and supported by the base plate 3a.

As shown in Fig. 4B, the electrolytic unit 5 has an electrolytic bath 52 to which water such as tap water or the like is supplied. At least a pair of electrodes 53a and 53b are disposed in the electrolytic bath 52, and by applying a voltage between these electrodes 53a and 53b, water is electrolyzed to generate electrolytic water containing active oxygen species.

Here, the active oxygen species is oxygen having higher oxidizing activity than normal oxygen and relevant materials thereto, and contain not only so-called narrowly-defined active oxygen such as superoxide anion, singlet oxygen, hydroxyl radical and hydrogen peroxide, but alsoso-called broadly-defined active oxygen such as ozone, hypochlorous acid, hypohalous acid, etc. The electrolytic bath 52 is fixed to one surface of the base plate 3a, and disposed in proximity to the element 41 disposed on the opposite side surface of the base plate 3a, so that electrolytic water containing active oxygen species can be immediately supplied to the element through the electrolytic water injection tube 51.

The electrodes 53a, 53b are constructed by two electrode plates each of which comprises a base of Ti (titan) and a coated layer of Ir (iridium), Pt (platinum).

By applying a voltage between the electrodes 53a and 53b, hydrogen ion (H⁺) and hydroxide ion (OH⁻) in water react with each other according to the following reaction formula (1) at the cathode:

4H⁺ + 4e⁻ + (40H⁻) → 2H₂ + (40H⁻) ... (1)

Furthermore, water is electrolyzed according to the following reaction formula (2) at the anode:

2H₂O → 4H⁺ + O₂ + 4e⁻ ... (2)

At the same time, chlorine ion (Cl⁻) contained water reacts according to the following reaction formula (3), and chlorine (Cl₂) is generated:

2Cl⁻ → Cl₂ + 2e⁻ ... (3)

Furthermore, Cl₂ thus generated reacts with water and hypochlorous acid (HC10) and hydrogen chloride (HCl) are generated:

Cl₂ + H₂O → HClO + HCl ... (4)

In this construction, by supplying current between the electrodes 53a and 53b, active oxygen species such as hypochlorous acid having strong sterilizing force, etc., and the electrolytic water containing this active oxygen species is supplied to the element 41. Air is passed through the element under the above state and virus, etc. floating in the air passing through the element 41 are inactivated (decomposed, etc.) by the electrolytic water to thereby filter the air, and further breeding of various bacteria, etc. in the element 41 itself can be prevented.

Furthermore, gaseous materials which cause odor, etc. in the air can be dissolved in the electrolytic water or react with the active oxygen species such as hypochlorous acid, etc. contained in the electrolytic water while passing through the element 41, whereby these materials are removed from the air and thus the air can be deodorized by the element 41.

Still furthermore, when current having a predetermined current density (for example, 20mA/cm² or the like) is supplied between the electrodes 53a and 53b, electrolytic water containing active oxygen species of a predetermined concentration (for example, free residual chlorine concentration of 1mg/l or the like) can be generated by electrolysis of water. Furthermore, by changing this current value, the concentration of the active oxygen species in the electrolytic water can be varied, and as a specific example, when the current value is reduced, the concentration of hydrochlorous acid in the electrolytic water can be increased.

Furthermore, as shown in Fig. 5, a tap water adjusting portion 6 having a connection pipe 50, the tap water control valve 46 and the check valve 47 is connected to the upstream side of the electrolytic unit 5. The check valve 47 is connected through a connector 59 to a tap water injection pipe 48 which is connected to a water source such as a water supply pipe (not shown) or the like. The tap water control valve 46 is disposed at the downstream side of the check valve 47. The opening/closing operation and the valve opening degree of the tap water control valve 46 are adjusted under the control of the electrical equipment board 40. The connection pipe 50 inter communicating with the water tap control valve 46 extends to the electrolytic unit 5, and water whose amount corresponds to the opening degree of the tap water control valve 46 is supplied to the electrolytic unit 5.

In this construction, the tap water injection pipe 48 is disposed so as to extend along the connection pipe 35 and the drainpipe 27a passing through the cut-out portion 20f to the neighborhood of the housing 20, and connected to the tap water adjusting portion 6 of the air filtering unit 3 through the connector 59. Therefore, when the indoor unit 2 is embedded and mounted in the ceiling, the connection works for the tap water injection pipe 48, the connection pipe 35, the drain pipe 27a, etc. can be performed in a lump, and the labor imposed on the setting work can be reduced.

Here, city water (tap water), water stocked in a water supply tank or the like may be used as the water source which is located at the upstream side of the tap water injection pipe 48 and supplies water such as tap water or the like. Furthermore, the water stocked in the water supply tank or the like may be water containing ion material such as chlorine ions or the like in advance such as tap water or the like, or water containing a small concentration of ion materials such as well water or the like. In this embodiment, these kinds of water are generically named as water.

The electrolytic water supplied to the air filtering portion 4 from the electrolytic unit 5 filters (inactivates, decomposes, deodorizes, sterilizes or the like) air in the element 41 of the air filtering portion 4, and then stocked in the electrolytic water tray 43. The water stocked in the electrolytic water tray 43 is passed through the drain pipe 44 to the drain tray (electrolytic water receiver) 62. This drain tray 62 is a tray for stocking the electrolytic water supplied to the air filtering portion 4, and is formed of a material having low reactivity to the electrolytic water (for example, foamed polystyrene). Furthermore, the drain tray 62 is provided at a position lower than the electrolytic water tray 43 in the height direction, and the electrolytic water stocked in the electrolytic water tray 43 is discharged to the drain tray 62 by the difference in height between the electrolytic water tray 43 and the drain tray 62, and stocked in the drain tray 62.

The drain tray 62 is provided with the drain pump 63 and the float switch 64 for detecting the electrolytic water stocked in the drain tray 62. The electrolytic water stocked in the drain tray 62 is pumped up through the suction nozzle 63A of the drain pump 63 by the operation of the float switch 64. The drain pipe 65 of the drain pump 63 is connected to the drain pipe 27a at a connection portion (confluent portion) 66 formed at the downstream side of the drain pump 27, and the electrolytic water pumped by the drain pump 63 is confluent with drain water at the connection portion 66 and discharged through the drain pipe 27a to the outside of the indoor unit 2.

According to this construction, the electrolytic water supplied from the electrolytic unit 5 to the air filtering portion 4 is discharged to the outside of the indoor unit 2 without passing through the drain pan 24, and thus the drain pan 24 and the indoor heat exchanger located in the drain pan 24 can be prevented from being corroded by the electrolytic water. Accordingly, the drain pan 24 and the steel pipes and fins of the indoor heat exchanger 21 are not required to be subjected to antiseptic and corrosion-proof treatments, and thus the manufacturing cost can be reduced.

Furthermore, only one drain pipe 27a is provided so as to extend to the outside of the indoor unit 2, so that the piping construction can be simplified and also the construction cost can be reduced. Furthermore, the electrolytic water is discharged through the drainpipe 27a, and thus occurrence of various kinds of bacteria, etc. in the drain pipe 27a can be prevented, and the inside of the drain pipe 27a can be kept clean.

Still furthermore, the connection portion 66 is provided at the downstream side of the maximum position H in the height direction of the drain pipe 27a and at a position lower than the position H. According to this construction, even when the electrolytic water is made confluent with the drain water at the connection portion 6, and then discharged to the outside of the indoor unit 2 through the drain pipe 27a, the connection portion 66 is provided at the position lower than the maximum position in the height direction of the drain pipe 27a, and thus the electrolytic water in the drain pipe 27a can be prevented from flowing back into the drain pan 24. Accordingly, the electrolytic water can be prevented from being stocked in the drain pan 24, so that the drain pan 24 and the indoor heat exchanger 21 located in the drain pan 24 can be prevented frombeing corroded.

The electrical equipment board 40 is equipped with CPU (not shown), a control program executed by CPU, ROM for storing control data associated with the control program, etc., and RAM for temporarily storing programs executed by CPU and various kinds of data. CPU performs various kinds of control such as the control of the current supply to the electrodes 53a, 53b (see Fig. 4B) in the electrolytic unit 5, the control of the opening degree of the tap water control valve 46, the driving control of the drain pump 63, etc. according to the control program stored in ROM For example, in order to generate electrolytic water of a predetermined concentration in the electrolytic unit 5, CPU makes current flow through the electrodes 53a and 53b by the current density corresponding to the concentration concerned. Furthermore, in order to supply the electrolytic unit 5 with water from the tap water injection pipe 48, CPU adjusts the opening degree of the tap water control valve 46.

Furthermore, CPU of the electrical equipment board 40 is connected to CPU of the controller 8 through a communication line or the like (not shown), and executes the above control according to an instruction input from the controller 8, for example, an instruction input in the remote controller. Accordingly, the electrolytic water can be supplied to the air filtering portion 4 interlockingly with or independently of the cooling/heating operation.

Furthermore, the float switch 64 is connected to the electrical equipment board 40 (CPU), and CPU executes various kinds of control such as the current supply control to the electrodes 53a, 53b in the electrolytic unit 5, the control of the opening degree of the tap water control valve 46, the driving control of the drain pump 63, etc.

Specifically, when a low water level is detected by the float switch 62, CPU opens the tap water control valve 46 to supply tap water or the like to the electrolytic unit 5. In this case, CPU increases the current density to be supplied to the electrodes 53, 53b on the basis of the opening degree of the tap water control valve 46. Furthermore, when a middle water level is detected by the float switch 62, CPU reduces the opening degree of the tap water control valve 46, and also reduces the current density to be supplied to the electrodes 53a, 53b on the basis of the opening degree. Furthermore, when a high water level is detected by the float switch 62, CPU closes the tap water control valve 46, and also stops the current supply to the electrodes 53a, 53b. Then, in order to discharge the electrolytic water in the drain tray 62 to the outside, the operation of the drain pump 63 is started. This drain pump 63 is stopped when the low water level is detected by the float switch 62.

According to this construction, since the amount of water to be supplied is controlled in accordance with the amount of electrolytic water stocked in the drain tray 62, the amount of electrolytic water which is needlessly discharged is reduced and thus the water can be saved. Furthermore, in accordance with the water amount supplied to the electrolytic unit 5, the current supply amount to the electrodes 53a, 53b is changed, so that the electrolytic water of a substantially fixed concentration can be supplied to the element 41 at all times irrespective of the supplied water amount, and the air filtering efficiency can be kept.

According to this embodiment, in the housing 20 are disposed the indoor heat exchanger 21 for air-conditioning air sucked by the air blowing fan 22, the drain pan for receiving drain water dropped from the indoor heat exchanger 21, the drain pump 27 for pumping drain water stocked in the drain pan 24 and discharging the drain water through the drain pipe 27a to the outside, and the element 41 which is disposed at the downstream side of the indoor heat exchanger 21 and into which electrolytic water containing active oxygen species is in filtrated and brought into contact with the sucked air to thereby filter the air. The electrolytic water dropped from the element 41 is guided to the drain tray 62 different from the drain pan 24, so that the electrolytic water is discharged to the outside of the indoor unit 2 without passing through the drain pan 24. Accordingly, the drain pan 24 and the indoor heat exchanger 21 located in the drain pan 24 are prevented from being corroded by the electrolytic water. Accordingly, the drain pan 24 and the steel pipe and fin of the indoor heat exchanger 21 are not required to be subjected to the antiseptic and corrosion-proof treatment, so that the manufacturing cost can be reduced.

Furthermore, the electrolytic water passing through the drain tray 62 is made confluent with the drain water at the downstream side of the drain pump 27. Therefore, only the drain pipe 27a serves as only one pipe provided so as to extend to the outside of the indoor unit 2, so that the piping construction can be simplified and the construction cost can be reduced. Furthermore, the electrolytic water is discharged through the drain pipe 27a, so that occurrence of various kinds of bacteria in the drain pipe 27a can be prevented and the inside of the drain pipe 27a can be kept clean.

Still furthermore, according to this embodiment, the drain pipe 27a is provided with the connection portion 66 at which the drain pipe 65 is connected to the drain pipe 27a and the drain water and the electrolytic water are confluent with each other. The connection portion 66 is located at the downstream side with respect to the maximum position H in the height direction of the drain pipe 27a and at a position lower than the maximum position H. Therefore, even in the construction that the electrolytic water is discharged through the drain pipe 27a to the outside of the indoor unit 2, the electrolytic water in the drain pipe 27a can be prevented from flowing back into the drain pan 24. Accordingly, electrolytic water can be prevented from being stocked in the drain pan 24, and thus the corrosion of the drain pan 24 and the indoor heat exchanger 21 located in the drain pan 24 can be prevented.

Furthermore, according to this embodiment, in the air filtering unit 3, the element 41 is fixed to one surface of the base plate 3a, and the electrolytic unit 5 for generating electrolytic water is provided to the other surface. Accordingly, by fixing the base plate 3a to the side plate 20a, the air filtering portion 4 and the electrolytic unit 5 can be easily secured to the housing. Furthermore, the element 41 and the electrolytic unit 5 are proximate to each other, and thus there is an advantage that the generated electrolytic water can be quickly supplied to the element 41. Furthermore, the drain tray 62 is provide to the other surface of the base plate 3a, and thus the drain tray 62 can be easily secured by fixing the base plate 30a to the side plate 20a.

Still furthermore, according to this embodiment, one air filtering unit 3 is secured to the indoor unit 2. Another air filtering unit 3 can be easily added to the indoor unit 2. In the construction of this embodiment, three air filtering units 3 can be secured at maximum.

That is, the three side plates 20a constituting the housing 20 have the knockout hole portions 20c, and the air filtering units can be provided to all the knockout hole portions 20c at these three places.

According to this construction, the number of the air filtering units to be secured to the indoor unit 2 can be changed in accordance with the required air filtering capability, and thus the air filtering capability conformed with the property of the room to be air-conditioned can be implemented. Accordingly, for example, in a hospital, a school or a place which the general public enters and leaves, a large number of air filtering units 3 are secured to the indoor unit to implement high air filtering performance. Furthermore, when the hermitically sealing performance of the room to be air-conditioned is relatively high and thus a sufficient effect can be attained by low air filtering capability, a small number of air filtering units 3 may be secured to the indoor unit 2. The adjustment of the air filtering capability can be easily implemented by a simple work of punching out the knockout hole portions 20c and securing the air filtering units 3 to the punched portions.

The present invention is not limited to the above embodiment, and various modifications may be made without departing from the subject matter of the present invention.

In the above embodiment, hypochlorous acid is generated as the active oxygen species. However, ozone (O₃) or hydrogen peroxide (H₂O₂ may be generated as active oxygen species. In this case, when platinum tantalum electrodes are used as the electrodes 53a,53b, active oxygen species can be highly efficiently and stably generated from water in which ion species are rare.

That is, by supplying current between the electrodes 53a and 53b, at the anode, the reactions represented by the following chemical formulas (5) to (7) occur, and ozone is generated.

2H₂O → 4H⁺ + O₂ + 4e⁻ ... (5)

3H₂O → O₃ + 6H⁺ + 6e⁻ ... (6)

2H₂O → O₃ + 4H⁺ + 4e⁻ ... (7)

Furthermore, at the cathode, the reactions represented by the following chemical formulas (8) and (9) occur, and O²⁻ generated through electrode action and H⁺ in solution are bonded with each other to generate hydrogen peroxide (H₂O₂) :

4H⁺ + 4e⁻ + (40H⁻) → 2H₂ + (40H⁻) ... (8)

O₂⁻ + e⁻ + 2H⁺ → H₂O₂ ... (9)

In this construction, by supplying current to the electrodes 53a and 53b, ozone (O₃) and hydrogen peroxide (H₂O₂) having strong sterilization power are generated, and electrolytic water containing ozone (O₃) and hydrogen peroxide (H₂O₂) can be generated. The concentration of ozone or hydrogen peroxide in this electrolytic water is adjusted to such a concentration that target virus, etc. can be inactivated, and by passing air through the gas-liquid contact member 53 supplied with the electrolytic water having this concentration, target virus, etc. floating in the air can be inactivated. Furthermore, when gaseous materials such as odor components, etc. pass through the element 41, these components are dissolved in the electrolytic or react with ozone or hydrogen peroxide in the electrolytic water, so that the materials are removed from the air and thus the air can be deodorized.

In the indoor unit 2, the same reaction can be induced even when water containing rare ion species (containing pure water, purified water, well water, some kinds of tap water, etc.) is used. That is, by adding halide (salt or the like) to water containing rare ion species, the same reactions as (3) and (4) are induced and active oxygen species can be achieved. That is, in this construction, the water is not limited to tap water added with a sufficient amount of chlorine compounds, but a sufficient air cleaning efficiency (inactivation, sterilization, deodorization, etc. of virus, etc.) can be exercised even when other types of water are used.

In this case, chemical compound (halide or the like) may be supplied to water introduced into the electrolytic bath 52. For example, a chemical compound supply device for supplying the chemical compound may be provided to the indoor unit 2. The chemical compound supply device may be designed so as to inject the chemical compound on the passage extending from the tap water injection pipe 48 to the electrolytic bath 52 or directly inject the chemical compound into the electrolytic bath 52, or the chemical compound having an adjusted concentration may be supplied to the indoor unit 2.

Here, salt or brine may be used as the chemical compound. For example, when the concentration of brine in the electrolytic bath 52 is adjusted to about 2 to 3% (weight percentage), electrolytic water (0.5 to 1%) containing hypochlorous acid or hydrogen peroxide can be generated by electrolyzing the brine in the electrolytic bath 52. According to this construction, even when the ion species in the water introduced into the electrolytic bath 52 is rare, the ion species can be increased by adding salt or brine, so that active oxygen species can be stably generated with high efficiency in the electrolysis of water.

Furthermore, in the above embodiment, the material constituting the drain tray 62 and the detailed construction such as the shape, etc. of the indoor heat exchanger 21 may be arbitrarily changed.

## Claims

1. An air conditioner including a heat exchanger (21) for air sucked by an air blower (22), a drain pan (24) for receiving drain water dropping from the heat exchanger (21), a drain pump (27) for pumping up drain water stocked in the drain pan (24) and discharging the drain water through a drain hose (27a) to the outside, a gas-liquid contact member (41) that is disposed at the downstream side of the heat exchanger (21) in an air flowing direction and supplied with electrolytic water containing active oxygen specifies so that the electrolytic water infiltrates into the gas-liquid contact member (41) and brought into contact with the air sucked by the air blower (22) to filter the air, and a housing (20) in which the heat exchanger, the drain pan, the drain pump and the gas-liquid contact member are mounted, wherein the electrolytic water dropped form the gas-liquid contact member is guided to an electrolytic water receiver (62) which is provided separately from the drain pan, and the electrolytic water passed through the electrolytic water receiver (62)is made confluent with the drain water at the downstream side of the drain pump (27).

2. The air conditioner according to claim 1, wherein the drain hose is equipped with a confluent portion (66) at which the drain water and the electrolytic water are confluent with each other, and the confluent portion (66) is provided so as to be located at the downstream side of the maximum position of the drain hose (27a) in the height direction and at a lower position than the maximum position.

3. The air conditioner according to claim 1 or 2, wherein an opening portion (20d) is formed in at least one surface of the housing, the gas-liquid contact member is fixed to one surface of a plate-shaped member (3a) which covers the opening portion (20d), and an electrolytic unit (5) for generating the electrolytic water is provided on the other surface of the plate-shaped member.

4. The air conditioner according to claim 3, wherein the electrolytic receiver (62) is provided to the other surface of the plate-shaped member.
